# EUROPEAN PATENT APPLICATION

(11) **EP 3 150 695 A1**
(43) Date of publication of application: **05.04.2017**
(21) Application number: 15799435.1
(22) Date of filing: 08.05.2015
(51) Int. Cl.: C12M 1/42

(54) **APPARATUS AND METHOD FOR MAGNETIC BEAD METHOD**

(30) Priority: 27.05.2014 CN 201410229179
(71) Applicant: Degree Of Freedom Scientific Machine Co., Ltd., Beijing 100176 (CN)
(72) Inventor: LIU, Xinzhi, Beijing 100176 (CN); ZHAO, Haifeng, Beijing 100176 (CN)
(74) Representative: Guella, Paolo
(86) International application number: PCT/CN2015/078627
(87) International publication number: WO 2015/180561

(57) **Abstract**

Provided is an apparatus for a magnetic bead method. The apparatus comprises a magnetic needle sheath, a magnetic needle, and a container. The magnetic needle sheath is provided with a main body and a bottom portion. An external surface of the bottom portion is provided with one or more vertically disposed blades. Also provided are the magnetic needle sheath and a method for processing magnetic particles in a liquid composition by using the apparatus or the magnetic needle sheath.

## Description

The present application claims priority of the Chinese patent application numbered 201410229179.8 entitled Device and Method for Use in a Magnetic Bead Method, filed on May 27, 2014. The disclosure of this application is incorporated by reference in its entirety.

### TECHNICAL FIELD

The invention relates to the field of biology and devices. Specifically, the invention provides a device for use in a magnetic bead method to treat magnetic particles in a liquid composition and extract bioactive substances, as well as a method for treating magnetic particles and extracting bioactive substances in a liquid composition using the device.

### BACKGROUND OF THE INVENTION

The gene detection has become a marker of the development of the biological industry, thereby requiring methods and apparatus for a high flux, automated nucleic acid extraction in the biological field to extract or detect various bioactive substances, such as cells, as well as proteins, nucleic acids, and the like. Nucleic acids are one of the most basic substances of the life and is widely present in all animals, plant cells and microorganisms. According to the chemical composition, the nucleic acids can be divided into ribonucleic acid (RNA) and deoxyribonucleic acid (DNA). As the PCR technology is applied in various fields, including disease diagnostic in medical field, transgenic detection in agriculture field, and other applications, method and apparatus for better efficacy and full-automatic bioactive substance extraction are needed.

Magnetic bead method is a technical developed in recent years for extracting bioactive substances, especially active macromolecules. Magnetic bead method utilizes magnetic beads which are capable of binding or specifically binding cells, proteins, or nucleic acids, and then, under the action of a magnetic field, isolated from active macromolecules in samples of blood, animal tissue, food, pathogenic microorganisms, and the like.

Modern magnetic bead methods include magnetic rod method (or 'magnetic needle method'), in which a magnetic needle or the magnetic rod is inserted into a sleeve, or a magnetic needle sleeve, so that the surface of the magnetic needle sleeve is magnetic, and then the sleeve is inserted into the blood, container for liquid samples such as animal tissue, food, pathogenic microorganisms, the particles that are capable of being magnetically attracted in the sample are concentrated on the surface of the sleeve through the magnetic field effect; when the magnetic rod is pulled away, the sheath loses magnetic field, and the magnetically attracted particle depart from the sheath. A magnetic rod method nucleic acid extractor generally comprises a plurality of channels, for example, 8, 16, 32, 96 channels, may handle multiple sets of magnetic needles and magnetic needle sleeves at the same time, and achieve a large flux and an automated treatment of a biological sample.

There is a need in the technical field for a more efficient device and method for use in a magnetic bead method to separate bioactive substances (such as RNA and DNA) in a liquid sample which can bind with magnetic beads and thus turning magnetic themselves.

### SUMMARY OF THE INVENTION

The present application provides a device for the use in a magnetic bead method to treat magnetic particles and extracting bioactive substances in a liquid composition with high efficacy and a method for treating magnetic particles in a liquid composition by using the device. The device and the method can be applied to clinical disease diagnosis, safe blood transfusion, forensic identification, environmental microbe detection and food safety, molecular biology studies, etc., for extraction and purification of bioactive substances, such as cells, proteins, and nucleic acids.

The present invention provides a device for use in a magnetic bead method, which can be used to treat magnetic particles in a liquid composition (solution). The device of the present invention comprises a magnetic needle sleeve, a magnetic needle, and a container, wherein said container may contain a solution comprising magnetic particles, said magnetic needle can be inserted into said magnetic needle sleeve, thereby generating a magnetic field on the outer surface of the magnetic needle sleeve, said magnetic needle sleeve can be inserted into the container, and when the magnetic needle is inserted into the magnetic needle sleeve, the outer surface of the magnetic needle sleeve generates a magnetic field so that the magnetic particles in the container can be adsorbed to the outer surface of the magnetic needle sleeve, and when the magnetic needle is pulled away from the magnetic needle sleeve, the magnetic field of the outer surface of the magnetic needle sleeve disappears, and the magnetic particles depart from the outer surface of the magnetic needle sleeve.

The magnetic needle sleeve is made of non-magnetic materials and cannot be magnetized, such as a non-metallic material. When no magnetic needle is inserted into it, the magnetic needle sleeve cannot adsorb magnetic particles in the magnetic field. The diameter of the magnetic needle sleeve is smaller than the diameter of the container of the device, so that the magnetic needle sleeve can be inserted into the container.

The magnetic needle sleeve has a sleeve body and a sleeve bottom, wherein the sleeve body is a hollow rod and the top is open, the sleeve bottom is a closed structure. The outer surface of the sleeve bottom has one or more vertically arranged blades.

In one aspect of the present invention, the outer surface of the bottom of the magnetic needle sleeve has a plurality of said blades. Preferably, the blades are uniformly distributed, such as uniformly and symmetrically distributed.

In one aspect of the present invention, the upper end of the blade is connected with the outer surface of the bottom of the magnetic needle sleeve. In a further aspect of the present invention, the inside end of the upper end of the blade is located at the center point of the bottom of the magnetic needle sleeve. In a yet further aspect of the present invention, the outside end of the upper end of the blade is lever with the outer edge of the bottom of the magnetic needle sleeve.

In one aspect of the present invention, the bottom edge of the blade inclines to the outside. Preferably, the angle of the inclination is less than 20 degrees, more preferably, less than 10 degrees, and most preferably less than 5 degrees.

In one aspect of the present invention, the bottom edge of the blade does not incline to the outside. For example, said bottom edge is a horizontal line.

In one aspect of the present invention, the lowest end of the blade extends downwards less than 5 mm than the lowest end point of the outer surface of the bottom of the magnetic needle sleeve, preferably less than 2 mm.

In a further aspect of the present invention, the bottom edge of the blade inclines to the outside, the horizontal position of the outside end of the blade extends downwards less than 5 mm than the horizontal position of the lowest end point of the outer surface of the bottom of the magnetic needle sleeve, preferably less than 2 mm.

In a further aspect of the present invention, the horizontal position of the inside end of the blade is the same as that of the lowest end point of the outer surface of the bottom of the magnetic needle sleeve, or the horizontal position of the inside end of the blade is lower than that of the lowest end point of the outer surface of the bottom of the magnetic needle sleeve.

In a further aspect of the present invention, the bottom edge of the blade is a horizontal line, the horizontal position thereof is the same as that of the lowest end point of the outer surface of the bottom of the magnetic needle sleeve, or the horizontal position thereof is less than 5 mm lower than the horizontal position of the lowest end point of the outer surface of the bottom of the magnetic needle sleeve, preferably less than 2 mm.

In one aspect of the present invention, the blade connecting section between each two adjacent blades has an arc-shaped surface that is recessed inwardly.

In one aspect of the present invention, the sleeve body is a hollow cylinder.

In one aspect of the present invention, the inner surface of the bottom of the needle sleeve is a conical surface or a flat surface.

In one aspect of the present invention, the entire body or the bottom end of the magnetic needle is magnetic. The magnetic part of the magnetic needle is a permanent magnet or an electromagnet.

In the present invention, the magnetic particles are magnetic beads or bioactive substances combined with the magnetic beads, for example, nucleic acids (DNA or RNA), proteins or cells, and the like.

The present invention provides a magnetic needle sleeve for use in a magnetic bead method. A magnetic needle can be inserted into said magnetic needle sleeve from the top of the sleeve body, thereby generating a magnetic field on the outer surface of the magnetic needle sleeve, said magnetic needle sleeve can be inserted into a container, and when the magnetic needle is inserted into the magnetic needle sleeve and generates a magnetic field at the outer surface of the magnetic needle sleeve, the magnetic particles in the container can be adsorbed to the outer surface of the magnetic needle sleeve, and when the magnetic needle is pulled away from the magnetic needle sleeve, the magnetic field of the outer surface of the magnetic needle sleeve disappears, and the magnetic particles depart from the outer surface of the magnetic needle sleeve.

The magnetic needle sleeve is made of non-magnetic materials and cannot be magnetized, such as a non-metallic material. When no magnetic needle is inserted into it, the magnetic needle sleeve cannot adsorb magnetic particles in the magnetic field. The diameter of the magnetic needle sleeve is smaller than the diameter of the container of the device, so that the magnetic needle sleeve can be inserted into the container.

The aforementioned magnetic needle sleeve of the present invention has the following characteristics.

In one aspect of the present invention, the magnetic needle sleeve has a sleeve body and a sleeve bottom, wherein the sleeve body is a hollow rod and the top is open, the sleeve bottom is a closed structure. The outer surface of the sleeve bottom has one or more vertically arranged blades.

In one aspect of the present invention, the outer surface of the bottom of the magnetic needle sleeve has a plurality of said blades. Preferably, the blades are uniformly distributed, such as uniformly and symmetrically distributed.

In one aspect of the present invention, the upper end of the blade is connected with the outer surface of the bottom of the magnetic needle sleeve. In a further aspect of the present invention, the inside end of the upper end of the blade is located at the center point of the bottom of the magnetic needle sleeve. In a yet further aspect of the present invention, the outside end of the upper end of the blade is lever with the outer edge of the bottom of the magnetic needle sleeve.

In one aspect of the present invention, the bottom edge of the blade inclines to the outside. Preferably, the angle of the inclination is less than 20 degrees, more preferably, less than 10 degrees, and most preferably less than 5 degrees.

In one aspect of the present invention, the bottom edge of the blade does not incline to the outside. For example, said bottom edge is a horizontal line.

In one aspect of the present invention, the lowest end of the blade extends downwards less than 5 mm than the lowest end point of the outer surface of the bottom of the magnetic needle sleeve, preferably less than 2 mm.

In a further aspect of the present invention, the bottom edge of the blade inclines to the outside, the horizontal position of the outside end of the blade extends downwards less than 5 mm than the horizontal position of the lowest end point of the outer surface of the bottom of the magnetic needle sleeve, preferably less than 2 mm.

In a further aspect of the present invention, the horizontal position of the inside end of the blade is the same as that of the lowest end point of the outer surface of the bottom of the magnetic needle sleeve, or the horizontal position of the inside end of the blade is lower than that of the lowest end point of the outer surface of the bottom of the magnetic needle sleeve.

In a further aspect of the present invention, the bottom edge of the blade is a horizontal line, the horizontal position thereof is the same as that of the lowest end point of the outer surface of the bottom of the magnetic needle sleeve, or the horizontal position thereof is less than 5 mm lower than the horizontal position of the lowest end point of the outer surface of the bottom of the magnetic needle sleeve, preferably less than 2 mm.

In one aspect of the present invention, the blade connecting section between each two adjacent blades has an arc-shaped surface that is recessed inwardly.

The present invention also provide a method for treating magnetic particles in a liquid composition using a device or a magnetic needle sleeve as described above, which comprises the following steps: a. inserting the magnetic needle sleeve into a container containing a liquid composition which comprises magnetic particles; b, inserting the magnetic needle into the magnetic needle sleeve, so that a magnetic field is generated on the surface of the magnetic needle sleeve, magnetic particles in the liquid composition are then adsorbed onto the surface of the magnetic needle sleeve under the effect of a magnetic field; c, pulling out the magnetic needle from the magnetic needle sleeve, the magnetic field outside the surface of the magnetic needle sleeve disappears, and the magnetic particles depart from the magnetic needle sleeve.

In one aspect of the invention, the method further includes a step of moving the magnetic needle to a designated site while keeping the magnetic needle in the state of being inserted in the magnetic needle sleeve. Thus, the magnetic particles are adsorbed on the surface of the magnetic needle sleeve under the action of a magnetic field and move along with the magnetic needle sleeve.

In the present invention, the magnetic particles are magnetic beads or bioactive substances combined with the magnetic beads, for example, nucleic acids (DNA or RNA), proteins or cells, and the like.

The device and the magnetic needle sleeve provided by the invention have blades arranged at the bottom of the magnetic needle sleeve, and the structural have the characteristics as described above. By means of these structure characteristics, the contact surface area between the bottom of the magnetic needle sleeve and the liquid is increased, and the actual adsorption surface of the magnetic needle sleeve is increased, so that the amount of magnetic particles adsorbed by the magnetic needle sleeve is increased, and magnetic particles can be adsorbed in a more complete way. Magnetic bead method, particularly magnetic rod method, includes a step of moving the magnetic needle to a designated site while keeping the magnetic needle in the state of being inserted in the magnetic needle sleeve, the magnetic particles are adsorbed on the surface of the magnetic needle sleeve under the action of a magnetic field and move along with the magnetic needle sleeve. The bottom of the magnetic needle sleeve is provided with the blades, and the liquid of the solution containing the magnetic particles to be treated can form a liquid flow more easily when the magnetic needle sleeve moves up and down, especially when the needle sleeve is moving up and leaving the surface of the solution, so that the liquid has more channels to flow back to the container in a more smoothly way. Therefore, the phenomenon that the sample is splashed and the pollution is generated during the extraction and treatment of bioactive substances is reduced or even prevented. The structure of the magnetic needle sleeve provided by the invention can greatly reduce the lost (falling off) of the magnetic particles clusters condensed at the bottom of the needle sleeve during the transfer process, in particular, when a magnetic needle sleeve that adsorbs magnetic particles is moved up and leave the liquid in the container. In the device and method of the present invention, the magnetic particles in the composition in the solution can more effectively and more thoroughly adsorbed, thus the yield of the sampling is increased and the stability of the sample separation operation is ensured. More importantly, the inhibition of on the PCR reaction by the treated sample can be greatly avoided, and in the the fluorescence detection method, unwanted fluorescence signal is also greatly avoided, so that the sensitivity of the detection method is improved. In addition, the device and the magnetic needle sleeve provided by the invention can more effectively crush the cell debris in the biological sample, mixtures of fats and proteins because liquid vortex can be formed during stirring, so that the sample is fully mixed, thus, the bioactive molecules required in the sample are well separated and obtained.

### BRIEF DESCRIPTION OF THE DRAWINGS

FIG. 1 is a schematic diagram of a device according to the present invention used in a magnetic bead method for treating magnetic particles in a solution.
FIG. 2 is a cross-sectional view of A-A cross-sectional view of the device shown in FIG. 1.
FIG. 3 is a schematic view of a first direction of a magnetic needle sleeve of a preferred embodiment of the present invention.
FIG. 4 is a cross-sectional schematic view of B-B cross-sectional view of the device shown in FIG. 3.
FIG. 5 is a schematic view of another direction of a magnetic needle sleeve of a preferred embodiment of the present invention.
FIG. 6 is a schematic view of a magnetic needle sleeve of another preferred embodiment of the present invention.

### Reference signs for the appended drawings

1 Magnetic needle sleeve
11 sleeve body
12 sleeve bottom
121 blade
1211 upper end of the blade
1212 bottom edge of the blade
1213 blade connecting section
2 Magnetic needle
3 Container

### DETAILED DESCRIPTION OF THE INVENTION

Here below, the present invention is to be described at length in conjunction with the appended drawings for a better understanding and description of the present invention.

The invention provides a device for used in the biological field, such as a magnetic rod method or a magnetic bead method, which can be used to treat samples, in particular, magnetic particles in a liquid sample, for example, are used for mixing, separating biological active substances capable of being combined with magnetic beads and the like.

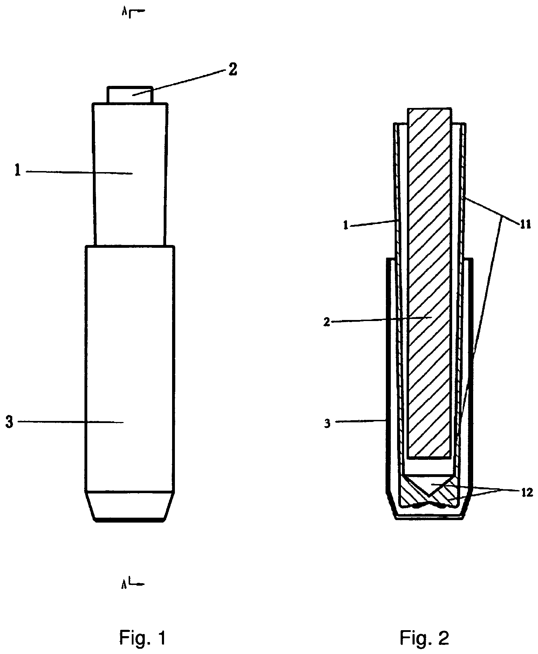

FIG. 1 is a schematic diagram of a device according to the present invention used in a magnetic bead method for treating magnetic particles in a solution. FIG. 2 is a cross-sectional view of A-A cross-sectional view of the device shown in FIG. 1. As shown in the figures, the device comprises a magnetic needle sleeve 1, a magnetic needle 2, and a container 3. Said container may contain a solution comprising magnetic particles. The magnetic particles can be magnetic beads or biological active substances combined with magnetic beads. The solutions may be various known liquid or liquid composition systems in which bioactive substances or magnetic beads can be dissolved or suspended. Bioactive substances combined with the magnetic beads include nucleic acids (DNA and/or RNA that can be specifically combined with the magnetic beads), proteins or cells, and the like. Said magnetic needle is an assembly unit which is magnetic and be able to generate a magnetic field. The magnetic needle can be inserted into a magnetic needle sleeve, thereby generating a magnetic field on the outer surface of the magnetic needle sleeve, while when the magnetic needle is lifted out from the sleeve, the magnetic field outside said magnetic needle sleeve disappears. The magnetic sleeve can be inserted into the container, and when the magnetic needle is inserted into the magnetic needle sleeve, the outer surface of the magnetic needle sleeve generates a magnetic field so that the magnetic particles in the container can be adsorbed to the outer surface of the magnetic needle sleeve, thus move along with the magnetic needle sleeve; and when the magnetic needle is pulled away from the magnetic needle sleeve, the magnetic field of the outer surface of the magnetic needle sleeve disappears, and the magnetic particles depart from the outer surface of the magnetic needle sleeve.

The magnetic needle sleeve is of a hollow rod structure, and the cross section of the magnetic needle sleeve is generally circular, the top is opened, which allows the magnetic needle to insert. The bottom of the magnetic needle is of a closed structure. In other embodiments of the invention, the cross-section of the magnetic needle sleeve may also be non-circular, which can be such as square or diamond. The magnetic needle sleeve is made of materials which is non-magnetic, and may not be magnetized, such as a non-metallic material, for example a plastic or glass. When no magnetic needle is inserted into it, the magnetic needle sleeve cannot adsorb magnetic particles through a magnetic field. The diameter of the magnetic needle sleeve is smaller than the diameter of the container of the device, so that the magnetic needle sleeve can be inserted into the container.

The magnetic needle can be vertically inserted and pulled away from the magnetic needle sleeve. The whole part or the bottom end of the magnetic needle (ie, the end inserts into the magnetic needle sleeve) has magnetism. When the magnetic needle is inserted, contacts or approaches the bottom of the magnetic needle sleeve, a magnetic field can be generated outside the magnetic needle sleeve, and when the magnetic needle is disengaged, the magnetic needle is pulled out or leaves the bottom of the casing pipe, the magnetic field generated outside the magnetic needle sleeve disappears, so that the magnetic beads or the biological sample with the magnetic beads in the magnetic needle sleeve outer solution can be adsorbed and separated by the magnetic needle sleeve. The magnetic part of the magnetic needle can be a permanent magnet and can also be a electromagnet capable of generating magnetism through electromagnetic action.

The container has a cavity with an open top. The cavity may be various shape and size suitable for containing solutions comprising magnetic micro particles and for the magnetic needle sleeve to insert, such as holes, grooves, and the like.

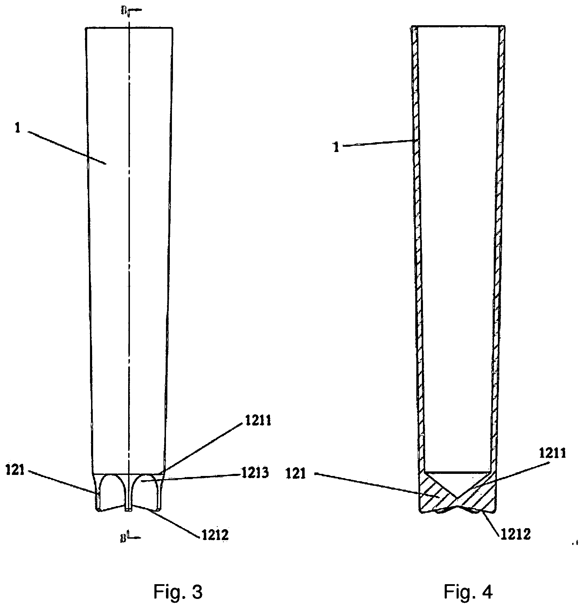

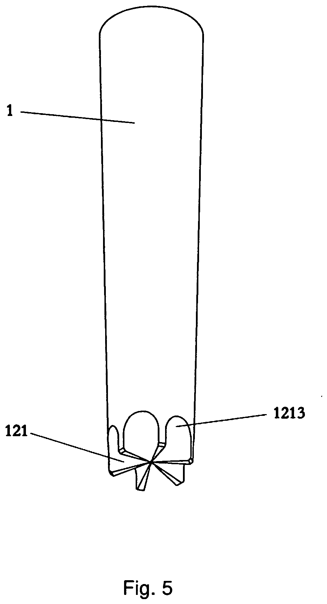

FIG. 3 is a schematic view of a first direction of a magnetic needle sleeve of a preferred embodiment of the present invention. FIG. 4 is a cross-sectional schematic view of B-B cross-sectional view of the device shown in FIG. 3. FIG. 5 is a schematic view of another direction of a magnetic needle sleeve of a preferred embodiment of the present invention.

As shown in the figures, as the preferred embodiment of the present invention, the magnetic needle sleeve is of a hollow rod structure, and the cross section is generally circular, the top is opened, which allows the magnetic needle to insert. The bottom of the magnetic needle is of a closed structure. In other embodiments of the invention, the cross-section of the magnetic needle sleeve may also be non-circular, which can be such as square or diamond. The magnetic needle sleeve is made of materials which is non-magnetic, and may not be magnetized, such as a non-metallic material, for example a plastic or glass. When no magnetic needle is inserted into it, the magnetic needle sleeve cannot adsorb magnetic particles through a magnetic field. The selection of the material used and the thickness of the magnetic needle sleeve is to not weaken the magnetic field or only slightly affect the magnetic field, and to ensure that a magnetic field strong enough to absorb magnetic beads or a biological sample binding with magnetic beads can be generated outside the magnetic needle sleeve when the magnetic needle is inserted into the sleeve. The diameter of the magnetic needle sleeve is smaller than the diameter of the container of the device, so that the magnetic needle sleeve can be inserted into the container, and there should be enough distance between the wall of the container and the sleeve so that to prevent the formation of the liquid ring.

As shown in FIG. 2 and FIG. 3-5, as the preferred embodiment of the present invention, the magnetic needle sleeve includes the sleeve body 11 and the sleeve bottom 12. The sleeve body is of a hollow cylindric structure, in which the diameter of the bottom surface can be slightly smaller than the diameter of the top surface (which forms a circular truncated cone body) or the bottom surface diameter is the same as the top surface diameter (which forms a cylinder).

The bottom of the magnetic needle sleeve is of a closed structure. The inner surface of the bottom of the magnetic needle sleeve is generally adapted to the shape of the bottom of the magnetic needle, in order to facilitate the a magnetic needle to insert, contact or get close to the bottom of the magnetic needle sleeve. In one aspect of the invention, the bottom of the magnetic needle sleeve has a conical inner surface. In another aspect of the invention, the bottom of the magnetic needle sleeve has a flat inner surface, such as a circular planar bottom. In other embodiments of the present invention, the bottom may also have other shapes of inner surfaces, such as raised hemispheres, etc..

The outer surface of the bottom of the magnetic needle sleeve is generally adapted to the shape of the bottom of the container, in order to facilitate the magnetic needle sleeve to insert, contact or get close to the bottom of the container. For example, the bottom of the magnetic needle sleeve has a flat outer surface.

The outer surface of the bottom of the magnetic needle sleeve of the present invention has a vertically disposed blade 121. As shown in FIGs. 2 and 3-5, the outer surface of the bottom of the magnetic needle sleeve of the device is provided with one or more blades 121, and the direction as which the blades are arranged is the same as the axial direction of the rod body of the magnetic needle sleeve. The working condition of the equipment of the present invention is that the magnetic needle, the magnetic needle sleeve and the container are arranged vertically, so that the magnetic needle sleeve can be vertically inserted or pull away from the container. Thus, in the present invention, the setting direction of the blade is referred to be vertical. For example, in the illustrated embodiment, the magnetic needle sleeve body is a cylinder, the bottom has a conical inner surface, as such, the phrase that the blades are arranged vertically refers to that the blades are arranged along the central axis direction of the cylinder body of the magnetic needle sleeve, i.e., perpendicular to the conical bottom of the inner surface of the bottom.

As shown in FIGs. 2 and 3-5, the upper end 1211 of the blade 121 is connected with the outer surface of the bottom of the magnetic needle sleeve, the inside end of the upper end of the blade is located at the center point of the bottom of the magnetic needle sleeve, the outside end of the upper end of the blade is lever with the outer edge of the bottom of the magnetic needle sleeve. In other embodiments of the present invention, the outside end of the upper end of the blade may not be lever with the outer edge of the bottom of the magnetic needle sleeve. For example, the outside end of the upper end of the blade is shorter or longer than the outer edge of the bottom of the magnetic needle sleeve.

In one aspect of the present invention, the bottom edge 1212 of the blade inclines to the outside, i.e., in the vertical direction, the horizontal position of the inner end point of the bottom edge is higher than the outer end point. In a further aspect of the present invention, the inclination angle (the angle between the horizontal plane and the straight line connecting the inner end point and the outer end point of the bottom edge) is less than 20 degrees, more preferably, less than 10 degrees, and most preferably less than 5 degrees. To have the bottom edge of the blade incline to the outside, it increases the the area of the bottom of the magnetic needle sleeve which for the absorbing the magnetic particles, therefore the working efficiency is improved. In the preferred embodiment of the present invention shown in FIGs. 3-5, the bottom edge 1212 of the blade is an inclined straight line that inclines outwards. In other embodiments of the invention, the bottom edge of the blade may be other shapes, such as a sawtooth or a wavy shape, or the like.

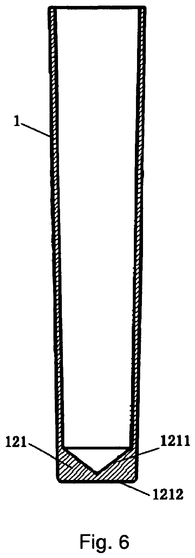

In the other aspects of the present invention, the bottom edge (1212) of the blade (121) may not incline to the outside. FIG 6 shows another preferred embodiment of the present invention, in which the bottom edge is a horizontal line.

In the device of the present invention, the blades at the bottom of the magnetic needle sleeve extend downwardly and the length of the extension is set to be, when the magnetic needle sleeve is inserted into the container and the magnetic needle is inserted into the magnetic needle sleeve, the magnetic needle can generate a magnetic field at the bottom of the magnetic needle sleeve (including the blades) which can effectively act on the magnetic particles in the container, especially on the magnetic particles at the bottom of the container. In one aspect of the invention, the lowermost end of each blade extends downwards relative to the lowest end point of the outer surface of the bottom of the magnetic needle sleeve is smaller than 5 mm, preferably less than 2 mm.

In one aspect of the present invention, the bottom edge 1212 of the blade inclines to the outside, the horizontal position of the outside end of the blade extends downwards less than 5 mm than the horizontal position of the lowest end point of the outer surface of the bottom of the magnetic needle sleeve, preferably less than 2 mm. In one aspect, the horizontal position of the inside end of the blade is the same as that of the lowest end point of the outer surface of the bottom of the magnetic needle sleeve (i.e., the inside end of the blade is connected with the lowest end point of the outer surface of the bottom of the magnetic needle sleeve), or the horizontal position of the inside end of the blade is lower than that of the lowest end point of the outer surface of the bottom of the magnetic needle sleeve.

In one aspect of the present invention, the bottom edge of the blade is a horizontal line, the horizontal position thereof is the same as that of the lowest end point of the outer surface of the bottom of the magnetic needle sleeve. In another aspect of the present invention, the horizontal position thereof is less than 5 mm lower than the horizontal position of the lowest end point of the outer surface of the bottom of the magnetic needle sleeve, preferably less than 2 mm.

In the device of the present invention, the blades at the bottom of the magnetic needle sleeve can be triangular, trapezoidal or other various suitable shapes, such as the left and right side edges thereof may be parallel or non-parallel straight lines. The side edges may also be non-linear, such as in waveline or zigzag shape.

In the embodiments as shown in the FIGs, the number of blades is six and uniformly distributed symmetrically. The side edge of the inner side of each blade is connected at the central point axis of the bottom of the magnetic needle sleeve. In other embodiments of the invention, the number of blades may be one, two or more than two. The distribution of the blades may be uniformly and may also be non-uniformly.

A magnetic bead method (including the magnetic bar method) for treating magnetic particles contained in a solution includes a step wherein the magnetic needle sleeves vibrate up and down in the solution containing in a container so as to lysis, rinse and purify the biological sample to be treated. In addition, the method further comprises the step of moving the magnetic needle to a designated place under a state that the magnetic needle is kept being inserted into the magnetic needle sleeve. At these steps, the magnetic substance is adsorbed on the outer surface of the magnetic needle sleeve, and moves up and down along with the magnetic needle sleeve, the liquid contains the relative magnetic particles flows against the magnetic articles and the outer surface of the magnetic needle sleeve. As the bottom of the magnetic needle sleeve is provided with the blades, the solution containing the magnetic particles to be treated can form a liquid flow more easily when the magnetic needle sleeve moves up and down, especially when the needle sleeve is moving up and leaving the surface of the solution, so that the liquid has more channels to flow back to the container in a more smoothly way. Therefore, the phenomenon that the sample is splashed and the pollution is generated during the extraction and treatment of bioactive substances is reduced or even prevented. In one aspect of the present invention, the blade connection 1213 between adjacent blades has an arc-shaped surface that is recessed inwardly. As such, the liquid containing the solution to be treated can flow more smoothly, and inhibit the sample splashing and generation of pollution more efficiently.

The present invention also provide a method for treating magnetic particles in a liquid composition using a device or a magnetic needle sleeve as described above, which comprises the following steps:
a. inserting the magnetic needle sleeve into a container containing a liquid composition which comprises magnetic particles;
b. inserting the magnetic needle into the magnetic needle sleeve, so that a magnetic field is generated on the surface of the magnetic needle sleeve, magnetic particles in the liquid composition are then adsorbed onto the surface of the magnetic needle sleeve under the effect of a magnetic field;
c. pulling out the magnetic needle from the magnetic needle sleeve, the magnetic field outside the surface of the magnetic needle sleeve disappears, and the magnetic particles depart from the magnetic needle sleeve.

In one aspect of the invention, the method further includes a step of moving the magnetic needle to a designated site while keeping the magnetic needle in the state of being inserted in the magnetic needle sleeve. Thus, the magnetic particles are adsorbed on the surface of the magnetic needle sleeve under the action of a magnetic field and move along with the magnetic needle sleeve. This step is for the separation and transfer of magnetic particles in a solution.

In the present invention, the magnetic particles are magnetic beads or bioactive substances combined with the magnetic beads, for example, nucleic acids (DNA or RNA), proteins or cells, and the like.

The magnetic needle sleeve provided by the invention increases the contact surface area between the bottom of the magnetic needle sleeve and the liquid is increased, the amount of magnetic particles adsorbed by the magnetic needle sleeve is increased, and magnetic particles can be adsorbed in a more complete way. The structure of the magnetic needle sleeve provided by the invention can greatly reduce the lost (falling off) of the magnetic particles clusters condensed at the bottom of the needle sleeve during the transfer process, in particular, when a magnetic needle sleeve that adsorbs magnetic particles is moved up and leave the liquid in the container. In the device and method of the present invention, the magnetic particles in the composition in the solution can be more effectively and more thoroughly adsorbed, thus the yield of the sampling is increased and the stability of the sample separation operation is ensured. More importantly, the inhibition of on the PCR reaction by the treated sample can be greatly avoided, and in the the fluorescence detection method, unwanted fluorescence signal is also greatly avoided, so that the sensitivity of the detection method is improved.

Although the exemplary embodiments and their advantages have been described at length herein, it should be understood that various alternations, substitutions and modifications may be made to the embodiments without departing from the spirit of the present invention and the scope as defined by the appended claims. As for other examples, it may be easily appreciated by a person of ordinary skill in the art that the order of the process steps may be changed without departing from the scope of the present invention.

In addition, the scope, to which the present invention is applied, is not limited to the process, mechanism, manufacture, material composition, means, methods and steps described in the specific embodiments in the specification. According to the disclosure of the present invention, a person of ordinary skill in the art should readily appreciate from the disclosure of the present invention that the process, mechanism, manufacture, material composition, means, methods and steps currently existing or to be developed in future, which perform substantially the same functions or achieve substantially the same as that in the corresponding embodiments described in the present invention, may be applied according to the present invention. Therefore, it is intended that the scope of the appended claims of the present invention includes these process, mechanism, manufacture, material composition, means, methods or steps.

## Claims

1. A device for use in a magnetic bead method, comprising a magnetic needle sleeve (1), a magnetic needle (2), and a container (3), wherein said container can contain a solution comprising magnetic particles; said magnetic needle can be inserted into said magnetic needle sleeve, thereby generating a magnetic field at the outer surface of the magnetic needle sleeve; said magnetic needle sleeve can be inserted into the container, and when the magnetic needle is inserted into the magnetic needle sleeve, the outer surface of the magnetic needle sleeve generates a magnetic field so that the magnetic particles in the container can be adsorbed to the outer surface of the magnetic needle sleeve, and when the magnetic needle is pulled away from the magnetic needle sleeve, the magnetic field of the outer surface of the magnetic needle sleeve disappears, and the magnetic particles depart from the outer surface of the magnetic needle sleeve;
wherein the magnetic needle sleeve (1) has a sleeve body (11) and a sleeve bottom (12), wherein the sleeve body (11) is a hollow rod and the top is open; said bottom (12) is of a closed structure; the outer surface of the sleeve bottom has one or more vertically arranged blades.

2. The device of claim 1, wherein the outer surface of the bottom of the magnetic needle sleeve has a plurality of said blades, preferably, the blades are uniformly distributed.

3. The device of claim 1 or 2, wherein the upper end of the blade (1211) is connected with the outer surface of the bottom of the magnetic needle sleeve, preferably, the inside end of the upper end of the blade (1211) is located at the centre point of the bottom of the magnetic needle sleeve, more preferably, the outside end of the upper end of the blade (1211) is lever with the outer edge of the bottom of the magnetic needle sleeve.

4. The device of any one of claims 1-3, wherein the bottom edge (1212) of the blade (121) inclines to the outside, preferably, the inclination angle is less than 20 degrees, more preferably, less than 10 degrees, and most preferably less than 5 degrees.

5. The device of any one of claims 1-3, wherein the bottom edge (1212) of the blade (121) does not incline to the outside, preferably, said bottom edge is a horizontal line.

6. The device of any one of claims 1-5, wherein the lowest end of the blade extends downwards less than 5 mm than the lowest end point of the outer surface of the bottom of the magnetic needle sleeve, preferably less than 2 mm, for example,
wherein the bottom edge (1212) of the blade inclines to the outside, the horizontal position of the outside end of the blade extends downwards less than 5 mm than the horizontal position of the lowest end point of the outer surface of the bottom of the magnetic needle sleeve, preferably less than 2 mm; or, wherein the horizontal position of the inside end of the blade is the same as that of the lowest end point of the outer surface of the bottom of the magnetic needle sleeve, or the horizontal position of the inside end of the blade is lower than that of the lowest end point of the outer surface of the bottom of the magnetic needle sleeve;
or,
wherein said bottom edge (1212) of the blade is a horizontal line, the horizontal position thereof is the same as that of the lowest end point of the outer surface of the bottom of the magnetic needle sleeve, or the horizontal position thereof is less than 5 mm lower than the horizontal position of the lowest end point of the outer surface of the bottom of the magnetic needle sleeve, preferably less than 2 mm.

7. The device of claim 1, wherein the blade connecting section (1213) between each two adjacent blades has an arc-shaped surface that is recessed inwardly.

8. The device of claim 1, wherein the material of the magnetic needle sleeve is non-magnetic, preferably is a non-metallic material, such as plastic or glass.

9. A magnetic needle sleeve for use in a magnetic bead method, comprising a sleeve body (11) and a sleeve bottom (12), wherein the sleeve body (11) is a hollow rod and the top is open; said bottom (12) is a closed structure; the outer surface of the sleeve bottom has one or more vertically arranged blades (121), wherein a magnetic needle can be inserted into the sleeve from the top of the sleeve body, thereby generating a magnetic field on the outer surface of the magnetic needle sleeve, said magnetic needle sleeve can be inserted into a container, and when the magnetic needle is inserted into the magnetic needle sleeve, the outer surface of the magnetic needle sleeve generates a magnetic field so that the magnetic particles in the container can be adsorbed to the outer surface of the magnetic needle sleeve, and when the magnetic needle is pulled away from the magnetic needle sleeve, the magnetic field of the outer surface of the magnetic needle sleeve disappears, and the magnetic particles depart from the outer surface of the magnetic needle sleeve.

10. The magnetic needle sleeve of claim 9, wherein the outer surface of the bottom of the magnetic needle sleeve has a plurality of said blades, preferably, the blades are uniformly distributed.

11. The magnetic needle sleeve of claim 9 or 10, wherein the upper end of the blade (1211) is connected with the outer surface of the bottom of the magnetic needle sleeve, preferably, the inside end of the upper end of the blade (1211) is located at the center point of the bottom of the magnetic needle sleeve, more preferably, the outside end of the upper end of the blade (1211) is lever with the outer edge of the bottom of the magnetic needle sleeve.

12. The magnetic needle sleeve of any one of claims 9-11, wherein the bottom edge (1212) of the blade (121) inclines to the outside, preferably, the inclination angle is less than 20 degrees, more preferably, less than 10 degrees, and most preferably less than 5 degrees.

13. The magnetic needle sleeve of any one of claims 9-12, wherein the bottom edge (1212) of the blade (121) does not incline to the outside, preferably, said bottom edge is a horizontal line.

14. The magnetic needle sleeve of any one of claims 9-13, wherein the lowest end of the blade extends downwards less than 5 mm than the lowest end point of the outer surface of the bottom of the magnetic needle sleeve, preferably less than 2 mm,
for example,
wherein the bottom edge (1212) of the blade inclines to the outside, the horizontal position of the outside end of the blade extends downwards less than 5 mm than the horizontal position of the lowest end point of the outer surface of the bottom of the magnetic needle sleeve, preferably less than 2 mm; or, wherein the horizontal position of the inside end of the blade is the same as that of the lowest end point of the outer surface of the bottom of the magnetic needle sleeve, or the horizontal position of the inside end of the blade is lower than that of the lowest end point of the outer surface of the bottom of the magnetic needle sleeve;
or,
wherein said bottom edge (1212) of the blade is a horizontal line, the horizontal position thereof is the same as that of the lowest end point of the outer surface of the bottom of the magnetic needle sleeve, or the horizontal position thereof is less than 5 mm lower than the horizontal position of the lowest end point of the outer surface of the bottom of the magnetic needle sleeve, preferably less than 2 mm.

15. The magnetic needle sleeve of claim 9, wherein the blade connecting section (1213) between each two adjacent blades has an arc-shaped surface that is recessed inwardly.

16. The magnetic needle sleeve of claim 9, wherein the material of the magnetic needle sleeve is non-magnetic., preferably a non-metallic material, such as plastic or glass.

17. A method for treating magnetic particles in a liquid composition using a device of any one of claims 1-8 or a magnetic needle sleeve of any one of claims 9-16, comprises the following steps: a. inserting the magnetic needle sleeve into a container containing a liquid composition which comprises magnetic particles; b, inserting the magnetic needle into the magnetic needle sleeve, so that a magnetic field is generated on the surface of the magnetic needle sleeve, magnetic particles in the liquid composition are then adsorbed onto the surface of the magnetic needle sleeve under the effect of a magnetic field; c, pulling out the magnetic needle from the magnetic needle sleeve, the magnetic field outside the surface of the magnetic needle sleeve disappears, and the magnetic particles depart from the magnetic needle sleeve.
